(19) Europäisches Patentamt / European Patent Office / Office européen des brevets

(11) **EP 4 105 942 A1**

(12) **EUROPEAN PATENT APPLICATION**

(43) Date of publication:
**21.12.2022   Bulletin 2022/51**

(21) Application number: **21305812.6**

(22) Date of filing: **14.06.2021**

(51) International Patent Classification (IPC):
**G16H 50/20** (2018.01)        **G16H 50/30** (2018.01)
**A61B 5/11** (2006.01)

(52) Cooperative Patent Classification (CPC):
**G16H 50/20; A61B 5/1118; G16H 50/30;**
G16H 20/30; G16H 80/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ESSILOR INTERNATIONAL
94220 Charenton-Le-Pont (FR)**

(72) Inventors:
• **LE CAIN, Aurélie
  94000 Creteil (FR)**
• **AMIR, Bruno
  94100 Saint Maur Des Fosses (FR)**

(74) Representative: **Plasseraud IP
66, rue de la Chaussée d'Antin
75440 Paris Cedex 09 (FR)**

(54) **METHOD AND SYSTEM FOR DETERMINING A TARGET VALUE OF A PARAMETER MEASURED BY A PORTABLE DEVICE**

(57)    The invention relates to a method for determining a target value for a parameter over an upcoming time window comprising:
- providing (S1) a set of data including, for each preceding time window, the value reached by the parameter and the associated target value;
- selecting (S2) an appropriate time scale among at least two time scales;
- retrieving (S3) data relating to preceding time windows based on the time scale;
- calculating (S4) a success factor based on the comparison between the value reached by the parameter over one or more preceding time windows and the associated target value; and
- determining (S5) the target value based on the success factor and the value reached by the parameter over each preceding time window of the retrieved data.
    The parameter can be a number of steps measured by a portable pedometer-type device worn by an individual.

FIG. 2

## Description

### FIELD OF INVENTION

The invention relates to the field of determining a target value to be reached for a parameter.

The method and system described hereinafter are particularly suitable for determining a target number of steps or distance to be travelled for an individual practicing a physical activity and wearing a portable pedometer-type device.

### BACKGROUND OF THE INVENTION

Today, more and more people use new technologies to manage different aspects of daily life, such as financial expenses, diet, health or physical activity. Tools have thus been developed and are accessible to users, for instance via applications which can be downloaded on smartphones. The principle of these tools is to measure one or more parameters in order to inform the user so that the latter adapts his or her behavior accordingly, for example by modifying his or her diet or by reducing his or her financial expenses.

Furthermore, it is advantageous for these tools to determine, by means of algorithms, target values of the measured parameters which thus constitute objectives for the user. A quantified goal indeed represents an additional motivation for the user. The technical challenge for the tools and applications is to have an algorithm that meets the user's needs and that can adapt to different conditions, including changes in the user's lifestyle. In recent years, several solutions have been proposed to determine, in real time, realistic goals adapted to each user.

For example, a moving-window percentile rank algorithm consists of measuring an individual's physical activity over several days, classifying these days according to the number of steps and then calculating a new goal based on a percentile criterion. The application can thus suggest to the individual to reach, for the following day, a number of steps corresponding to the 60th percentile. However, such an algorithm has drawbacks since it makes no distinction between the measurement days and does not adapt to possible drastic changes in the user's daily life. Among the other solutions of the prior art, certain algorithms propose to calculate a linear moving weighted average. The advantage of such a solution is to attribute a weighting coefficient to each measurement day and therefore to be able to adapt the objective according to the user's daily life. On the other hand, the calculation of the linear moving weighted average does not take a change in an individual's daily behavior into account. In addition, this solution does not consider the failure or success of the user to achieve the goals set on the previous days.

The present invention seeks to improve the situation.

### SUMMARY OF THE INVENTION

The present invention concerns a computer-implemented method for determining a target value to be reached for a parameter over an upcoming time window comprising:

- providing a set of data relating to preceding time windows and including, for each preceding time window, the value reached by the parameter over the preceding time window and the target value associated with the preceding time window;
- selecting an appropriate time scale among at least two predetermined time scales;
- retrieving data relating to one or more preceding time windows based on the time scale;
- calculating a success factor based on the comparison between the value reached by the parameter over one or more preceding time windows and the associated target value; and
- determining the target value for the upcoming time window based on the success factor and the value reached by the parameter over each preceding time window of the retrieved data.

The success factor allows to consider the capacity of the parameter, on one or more preceding time windows, to reach the target value. The retrieving of data related to one or more preceding time windows selected according to the appropriate time scale allows to use only relevant data and in particular to take advantage of a possible periodic behavior in the variations of the parameter value.

The value of the parameter is for instance measured by a portable device adapted to be worn by an individual.

Such a portable device can be sensitive to motion.

Typically, the portable device is a pedometer-type device. The parameter quantifies the intensity or quality of an individual's physical activity. The parameter relates for instance to a number of steps, a distance traveled by an individual, a number of calories burned or the duration of the physical activity.

The portable device sensitive to motion can also be an inertial measurement device configured to measure motion data of a mobile, for example acceleration or angular velocity.

In such cases, the parameter is therefore measured by a portable device and allows to characterize, for the individual wearing the portable device, his or her physical activity, lifestyle or, more generally, his or her behavior to maintain his or her health. The determined target value then constitutes an objective or goal for the individual. The appropriate time scale allows for the individual's habits to be considered. The success factor then corresponds to the individual's past successes or failures in achieving his or her objectives or goals.

Advantageously, the success factor is calculated based on a profile of the individual depending on physiological characteristics or data relating to a regular physical activity or health of the individual.

The use of physiological data or the individual's habits allows the process to be personalized.

According to an embodiment, the selection of the appropriate time scale depends on the variations in the parameter value over one or more preceding time windows.

By selecting an appropriate time scale, only relevant data are retained. Indeed, the variations of the parameter value can have a periodic behavior. Similarly, the ability of the parameter to reach the target value can also be fluctuating. This is especially true when the parameter quantifies an individual's behavior, such as physical activity. Thus, in general, it is advantageous to determine the appropriate time scale according to the variations of the value of the parameter over one or more preceding time windows.

According to an embodiment, each time scale of the at least two predetermined time scales relates to a period of time. The selection of the appropriate time scale comprises:

- for each time scale of the at least two predetermined time scales:

  • selecting data relating to preceding time windows spaced from the upcoming time window by a multiple of the period of time to which the time scale relates;
  • calculating, for each pair of two consecutive preceding time windows of the selected data, a difference between the values reached by the parameter respectively over each preceding time window of the pair of two consecutives preceding time windows;
  • assigning to the time scale a stability score characterizing the one or more calculated differences; and

- selecting the appropriate time scale based on the respective stability scores of the time scales of the at least two predetermined time scales.

The stability score assigned to a time scale depends for example on a ratio of pair of two consecutive preceding time windows of the selected data for which the calculated difference is lower than or equal to a predetermined threshold.

In such an embodiment, the selection of the appropriate time scale can be based on the detection of a point event that changes the behavior of the parameter. When the parameter allows to quantify the intensity or the quality of the physical activity of an individual, the comparison of the value of the parameter from one preceding time window to the next allows to detect a noticeable change in the daily life of the individual when this change has an impact on the physical activity of the individual. Such an embodiment makes it possible to consider a move as well as a vacation.

According to an embodiment, each time scale of the at least two predetermined time scales relates to a period of time. The selection of the appropriate time scale comprises:

- for each time scale of the at least two predetermined time scales:

  • selecting data relating to one or more preceding time windows spaced from the upcoming time window by a multiple of the period of time to which the time scale relates;
  • calculating, for each preceding time window of the selected data, a difference between the value reached by the parameter over the preceding time window and the target value associated with the preceding time window;
  • assigning to the time scale a regularity score characterizing the one or more calculated differences; and

- selecting the appropriate time scale based on the respective regularity scores of the time scales of the at least two predetermined time scales.

The regularity score assigned to a time scale depends on a ratio of preceding time windows of the selected data for which the calculated difference is lower than or equal to a predetermined threshold.

As in the previous embodiment, the analysis of the variations in the parameter value with respect to the target value determined for one or more preceding time windows allows to detect a change in the performance of the parameter. Thus, when the parameter quantifies the physical intensity or quality of an individual, this embodiment makes it possible to detect a temporary inability to achieve the objectives or goals set by the method, for example during a convalescence.

According to an embodiment wherein each time scale of the at least two predetermined time scales relates to a period of time, the retrieved data relate to one or more preceding time windows spaced from the upcoming time window by a multiple of the period of time to which the selected time scale relates.

According to an embodiment, the determination of the target value for the upcoming time window comprises weighting of the value reached by the parameter over each preceding time window of the retrieved data so that the weighting coefficient of the value reached by the parameter over a given time window is greater than or equal to the weighting coefficient of the value reached by the parameter over a time window prior to the given time window.

Preferably, the target value is determined according to a linearly weighted moving average calculation.

As previously explained, the method relies on past data, namely the value reached by the parameter over one or more preceding time windows as well as the target value determined for each of these preceding time windows. However, it may be advantageous to give more importance to the most recent data. Thus, the weighting allows to take more recent data than older data. The determined target value is therefore more accurate and realistic.

According to an embodiment, the method further comprises:

- generating a time series including values of the parameter, the time series being updated with each new value reached by the parameter over a given time window; and
- generating by learning at least one predictive model of the value of the parameter for a given time window as a function of a time component characterizing the time window;

The target value for the upcoming time window is thus determined using the at least one predictive model.

Advantageously, the time series is segmented into a plurality of groups of time windows according to a criterion related to the respective time components of the time windows and a predictive model is generated by learning for each group of time windows. In such a case, the determination of the target value for the upcoming time window comprises:

- applying the criterion to the time component of the upcoming time window in order to determine the corresponding group of time windows; and
- using the predictive model associated with the group of time windows to determine the target value for the upcoming time window.

When the parameter relates to the physical activity of an individual, for example the number of steps or the distance covered over each preceding time window, a segmentation of the time series is particularly advantageous since the physical activity of an individual depends on external conditions like for example the season. Typically, the time series can be segmented into four groups so that each group corresponds to a season. This segmentation allows to individualize the data and to take potential drastic changes in the individual's daily life into account.

This principle is true more generally for any type of parameter whose value may depend on external conditions that may evolve over time, especially by cycle.

The use of a predictive model allows the results previously obtained by the method to be used to determine the next target value more quickly and efficiently.

The present invention also relates to a computer program comprising instructions for implementing the previous method, when the instructions are executed by at least one processor.

Finally, the present invention concerns a system for determining a target value to be reached for a parameter over an upcoming time window comprising:

- a communication module arranged to receive a set of data relating to a plurality of preceding time windows;
- a storage unit arranged to store the received set of data, the data relating to a preceding time window including the value reached by the parameter over the preceding time window and the associated target value; and
- a processing unit configured to:

  - select an appropriate time scale among at least two predetermined time scales;
  - retrieve, from the storage unit, data relating to one or more preceding time windows based on the time scale;
  - calculate a success factor based on the comparison between the value reached by the parameter over one or more preceding time windows and the associated target value; and
  - determine the target value for the upcoming time window based on the success factor and the value reached by the parameter over each preceding time window of the selected data.

Typically, the system further comprises a portable device adapted to be worn by an individual and arranged to measure the value of the parameter and to transmit the value to the communication module.

## BRIEF DESCRIPTION OF THE DRAWINGS

Other features and advantages of the invention will become apparent from the following description provided for indicative and non-limiting purposes, with reference to the accompanying drawings, wherein:

- **FIG.1** illustrates a system for determining a target value to be reached for a parameter according to the invention;
- **FIG. 2** illustrates a method for determining a target value to be reached for a parameter according to the invention; and
- **FIG. 3** illustrates a step of selecting an appropriate time scale of the method illustrated in **FIG. 2.**

## DETAILED DISCLOSURE

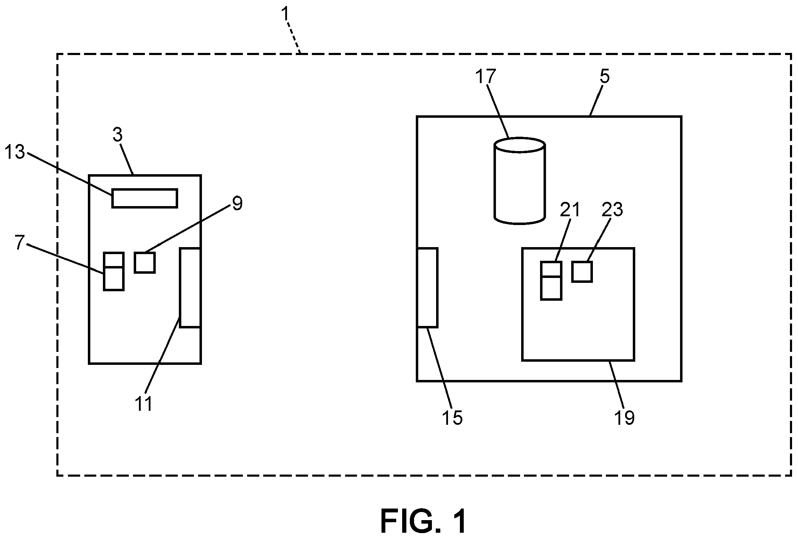

**FIG. 1** illustrates a system 1 for determining a target value to be reached for a parameter.

In the context of the invention, the parameter can be any parameter whose value varies over time and can be controlled, in particular by an individual. For instance, the parameter can be used to quantify an aspect of an individual's daily life such as his or her health or physical activity.

The system 1 is configured to measure the value of the parameter over time and to determine, at least based on the measurements, a target value that takes the form of a goal for the individual.

As illustrated in **FIG. 1,** the system 1 comprises a portable device 3, hereinafter device 3, and a server 5.

It should be understood here that the distinction between the system 1, the device 3 and the server 5 is purely functional and that any other configuration with the same functionality is possible.

In particular, the device 3 and the server 5 can form a single device or apparatus capable of implementing the functionalities not only of the device 3 but also of the server 5. **FIG. 1** simply relates to a particular embodiment in which certain functionalities are deported to a remote entity, namely the server 5.

The device 3 is arranged to measure the value of the parameter. The device 3 measures the value of the parameter for example in real time. Alternatively, the device 3 measures the value of the parameter at regular time intervals.

As previously explained, the device 3 illustrated in **FIG. 1** is a portable device. The portable device 3 is adapted to be worn by an individual. More generally, in the context of the invention, the device 3 is configured to measure a parameter relating to an individual's behavior or activity. This individual is, for example, the wearer of the portable device 3.

In a preferred embodiment, the portable device 3 is sensitive to motion.

The device 3 is for instance an inertial measurement unit (known under the acronym "IMU"). In such a case, the device 3 is an electronic device configured to measure motion data of a mobile, for example acceleration or angular velocity. The device 3 can also be a pedometer-type device. In such a case, the device 3 is configured to measure movement data relating to the walking of an individual. The device 3 can thus be worn by an individual and is configured to measure a number of steps or a distance traveled by the individual.

When the device 3 is a portable pedometer-type device sensitive to motion, the parameter measured by the device 3 may be any parameter quantifying the intensity or quality of an individual's physical activity. Therefore, the device 3 can also be configured to measure a number of calories burned or the duration of a physical activity.

Finally, the device 3 can also be configured to measure other parameters. The device 3 includes for instance an ambient light sensor (known under the acronym "ALS") configured to measure a parameter characterizing the time spent outside by an individual. The device 3 can also include an ultraviolet sensor configured to measure an amount of vitamin D produced by an individual's body. Still as an example, the device 3 can include a time-of-flight (known under the acronym "TOF") sensor configured to measure a parameter characterizing the time spent near to work by an individual.

The measurements collected by the device 3 are intended to be used to determine a target value of the parameter. This target value can thus be a target number of steps or distance to be covered for the individual wearing the device 3.

The device 3 comprises a memory 7, a processor 9, a communication module 11 and a screen 13.

The memory 7 is configured to store instructions whose implementation by the processor 9 results in the operation of the device 3. Furthermore, the memory 7 is also configured to store measurements collected by the device 3. The memory 7 may be a volatile or non-volatile memory, and the stored measurements may be erased after using these measurements to determine the target value to be reached by the parameter.

The communication module 11 is configured to transmit the measurements collected by the device 3 and stored in the memory 7 to the server 5. Of course, when the respective functionalities of the device 3 and the server 5 are implemented within the same device or apparatus, the communication module 11 is simply configured to route the measurements stored in the memory to a processing unit of that device or apparatus.

In the case of a remote server 5, it is known to the skilled person that there are many different types of data communication networks, for example cellular or non-cellular radio communication networks, and that, depending on the embodiment, the communication module 11 may integrate one or more communication sub-modules, for example for radiofrequency communication and be configured for the transmission and reception of radiofrequency signals, according to one or more technologies, such as TDMA, FDMA, OFDMA, CDMA, or one or more radiocommunication standards, such as GSM, EDGE, CDMA, UMTS, HSPA, LTE, LTE-A, WiFi (IEEE 802. 11) and WiMAX (IEEE 802.16), or their variants or evolutions, currently known or further developed, e.g. 5G.

The screen 13 is configured to display information that may be relevant to any individual, especially the individual wearing the device 3 in the embodiment in which the device is a portable pedometer-type device sensitive to motion. In this case, the screen 13 is configured to display the number of steps or the distance traveled. This information can be displayed in real time or at regular time intervals, for example at the end of the day.

Furthermore, when the functionalities of the device 3 and the server 5 are implemented in one and the same device or apparatus, the screen 13 can be configured to display the target value to be reached by the parameter.

According to a particular embodiment, the screen 13 may be a touch screen or a Human-Machine Interface configured to allow the individual wearing the device 3 to input information necessary for determining the target value to be reached by the parameter. The information can relate to a profile of the individual including physiological characteristics or data relating to a regular physical activity or health of the individual.

The server 5 is configured to determine the target value to be reached according to at least the measurements collected by the device 3.

As previously explained, the individual wearing the device 3 can also enter information via the screen 13. The information can also be transmitted by the communication module 13 to the server 5 for the determination of the target value.

In the embodiment illustrated in **FIG. 1,** the server 5 comprises a communication module 15, a storage unit 17 and a processing unit 19.

The communication module 15 is configured to receive measurements, and possibly profile information of the individual wearing the device 3, from the device 3 and more precisely from the communication module 11. In the particular case in which the functionalities of the device 3 and the server 5 are implemented within a single device or apparatus, the communication module 15 can form with the communication module 11 means for routing the measurements of the value of the parameter to the processing unit 19.

Conversely, in the case of a remote server 5, the communication module 15 of the server 5 can be similar to the communication module 11 of the device 3.

The storage unit 17 is arranged to store a set of data. In the context of the invention, the set of data stored in the storage unit 17 includes at least, for one or more time windows, the value reached by the parameter over each of these time windows and the target value determined for each time window. It should be understood here that a time window is a fixed time interval that can be expressed in a time unit. A time window is for example a day as clearly defined in a calendar. Of course, the skilled person understands that any other segmentation of time is possible to determine a plurality of time windows of fixed duration.

The storage unit 17 can be further configured to store additional information, such as information about the individual's profile and including physiological characteristics or data relating to the regular physical activity or health of the individual.

Furthermore, the storage unit 17 can also be configured to store a weighting coefficient associated with each time window and more specifically with each value reached by the parameter over a time window. Indeed, it may be relevant, in order to determine the target value to be reached by the parameter, to weight the values reached by the parameter over previous time windows. Typically, the weighting coefficients are determined so that the weighting coefficient of a given time window is greater than the weighting coefficient of a time window prior to the given time window. Such an embodiment is particularly suitable when the target value is determined according to a linearly weighted moving average calculation.

The processing unit 19 is configured to determine the target value to be reached by the parameter over an upcoming time window based on data stored in the storage unit 17.

As illustrated in **FIG. 1,** the processing unit 19 comprises a memory 21 and a processor 23.

The memory 21 is configured to store instructions whose implementation by the processor 23 results in the operation of the processing unit 19. The operation of the processing unit 19 results in the determination of the target value to be reached by the parameter for the upcoming time window. This target value can then be stored in the storage unit 17 in association with the upcoming time window.

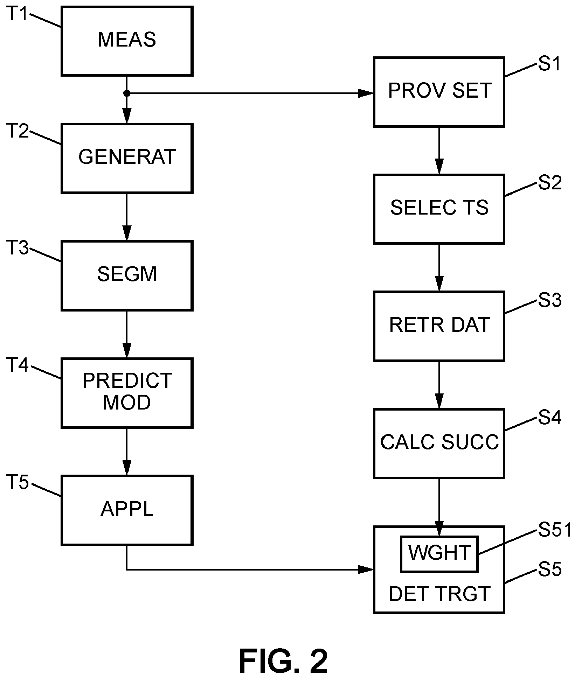

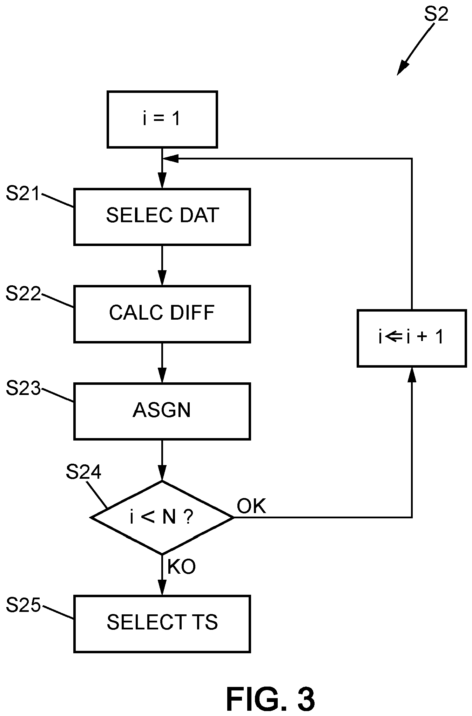

The functioning of the processing unit 19 will now be described in more detail below with reference to **FIG. 2** and **FIG. 3.**

In the general context of the invention, the method described hereinafter aims at determining a target value to be reached by a parameter over an upcoming time window. This target value is determined not only on the basis of measurements of the value of the parameter over one or more previous time windows but also on the basis of information such as success or failure in reaching the target value over one or more previous time windows. In a preferred application of the invention, the parameter is used to characterize the behavior or lifestyle of an individual and quantifies physical activity, diet or health of the individual. The target value determined for the upcoming time window thus constitutes a goal for the individual.

In the description of the method, which is the subject matter of the present invention, the parameter relates to a number of steps or a distance traveled by an individual measured by a portable pedometer-type device sensitive to motion, such as the device 3. As previously explained, the parameter can also be any parameter quantifying the intensity or quality of an individual's physical activity. The parameter can thus relate to a number of calories burned or the duration of a physical activity. The parameter can also relate to the time spent outside by an individual or an amount of vitamin D produced by an individual's body.

Of course, the same method can be implemented when the considered device 3 is an inertial measurement unit.

In a step S1, a set of data is provided. The set of data relates to preceding time windows and includes, for each preceding time window, the value reached by the parameter over the preceding time window and the target value associated with the preceding time window.

The set of data is for instance stored in the storage unit 17 and is provided to the processing unit 19 for the determination of the target value to be reached by the parameter over the upcoming time window.

Furthermore, the set of data may include additional data such as information relating to the profile of the individual and including physiological characteristics or data relating to the regular physical activity or health of the individual. As previously explained, the set of data also includes a weighting coefficient for each time window and more specifically for each value reached by the parameter over a time window.

In a step S2, an appropriate time scale is selected. More exactly, the appropriate time scale is selected among at least two predetermined time scales. The memory 21 of the processing unit 19 stores for instance a plurality of possible time scales among which the appropriate time scale can be selected.

A time scale is characterized by a period of time. The time scale makes it possible to select, among the preceding time windows, the relevant time windows, i.e. the time windows associated with data, for instance within the storage unit 17, which have to be used to determine the target value.

As previously explained, the method is specifically applied to the embodiment wherein the parameter relates to a number of steps or a distance traveled by an individual measured by the portable pedometer-type device 3 sensitive to motion. The value of the parameter depends on the physical activity of the individual. However, the physical activity depends on the habits of the individual and more generally on the organization of his or her daily life. Therefore, it is necessary to select an appropriate time scale to use the relevant data. Here, the time window corresponds to a day as defined in a calendar.

The time scale corresponds for example to a day, in which case the relevant data relate to the consecutive day or days preceding the upcoming day. Another possible time scale is a week. In such a case, the relevant data are not the data of the previous day but that of the same day of the previous week. Indeed, if the individual practices a physical activity every week only on Tuesday, the target number of steps or the target distance to be traveled for a Tuesday will not be determined according to the data relating to the day before or to the weekend. The data to be considered will indeed be the data relating to the Tuesday of the previous week(s). Of course, the skilled person understands that other time scales are possible, for example a time scale corresponding to one month.

The step S2 of selecting an appropriate time scale will now be described in more detail below with reference to **FIG. 3.**

According to an embodiment, the processing unit 19 tests one or more time scales to determine the most suitable for determining the target value. The principle is to analyze the variations in the parameter value for one or more preceding time windows. In the embodiment illustrated in **FIG. 3,** the memory 21 of the processing unit 19 stores a plurality of possible time scales, for instance N time scales, where N is an integer greater than or equal to 2. At the beginning of step S2, a local variable is initialized. This local variable scans the set of integers from 1 to N and allows to characterize each predetermined time scale. As previously explained, each time scale corresponds to a period of time. The following sub-steps are applied to each time scale, and more particularly to the associated period of time.

In a sub-step S21, the processing unit 19 selects data relating to one or more preceding time windows spaced from the upcoming time window by a multiple of the period of time to which the time scale relates are selected. The data are typically retrieved from the storage unit 17.

For instance, if the tested time scale corresponds to one week, the processing unit 19 selects at least the data corresponding to the same day of the previous week, thus the day spaced one week from the upcoming time window, i.e. the upcoming day. Of course, the processing unit 19 can also select data for the same day of several previous weeks, for example the day two weeks away from the upcoming day and the day three weeks away from the upcoming day.

Similarly, if the tested time scale is one day, the processing unit 19 can select data relating to the previous five days, since each of these days is separated from the upcoming day by a multiple of one day.

In a sub-step S22, the processing unit 19 calculates, for each preceding time window of the selected data, a difference between the value reached by the parameter over the preceding time window and the target value associated with the considered preceding time window.

As explained above, the storage unit 17 stores data relating to several preceding time windows. For each preceding time window, the storage unit 17 stores both the value reached by the parameter over that preceding time window and the target value determined for that preceding time window.

The difference is for example the absolute value of the difference between the reached value and the target value.

In the aforementioned example in which the time scale corresponds to one day and in which the processing unit 19 selects the data corresponding to the last five time windows, i.e. the last five days, the processing unit 19 calculates five differences, i.e. the difference between the value reached by the parameter and the target value for each of these last five days.

In a sub-step S23, the processing unit 19 assigns to the tested time scale a regularity score characterizing the one or more calculated differences. The regularity score assigned to the time scale depends for instance on a ratio of preceding time windows of the selected data for which the calculated difference is lower than or equal to a predetermined threshold.

For example, we now consider that the tested time scale corresponds to one week and that the processing unit 19 selected the data corresponding to the same day as the upcoming day but for the last three weeks. The processing unit 19 has thus calculated the difference between the value reached by the parameter and the associated target value for each of these three days. Furthermore, if the parameter corresponds to the number of steps, the predetermined threshold can be set to 1000. The regularity score can then correspond to the proportion of these three days for which there is less than 1000 steps difference between the number of steps taken by the individual and the target number of steps.

In a sub-step S24, the processing unit 19 determines, according to the value of the local variable, whether all the time scales have been tested. Otherwise, the following time scale is selected to be tested according to the sub-steps S21, S22 and S23 as explained previously.

Finally, in a sub-step S25, the processing unit 19 selects the appropriate time scale based on the respective regularity scores of the time scales of the at least two predetermined time scales.

If the regularity score assigned to a time scale depends on a ratio of preceding time windows of the selected data for which the calculated difference is lower than or equal to a predetermined threshold, the appropriate time scale is for instance the one with the highest score.

Of course, other embodiments are possible for the implementation of step S2. Advantageously, these other embodiments also depend on the variations of the value of the parameter over one or more preceding time windows. The embodiment detailed below can also be described with reference to **FIG. 3.**

Like the above-detailed embodiment, the memory 21 of the processing unit 19 can store a plurality of possible time scales, for instance N time scales, where N is an integer greater than or equal to 2. At the beginning of step S2, a local variable is initialized. This local variable scans the set of integers from 1 to N and allows to characterize each predetermined time scale. As previously explained, each time scale corresponds to a period of time. The following sub-steps are applied to each time scale, and more particularly to the associated period of time.

In the sub-step S21, the processing unit 19 selects data relating to preceding time windows spaced from the upcoming time window by a multiple of the period of time to which the time scale relates are selected. The data are typically retrieved from the storage unit 17.

In the sub-step S22, the processing unit 19 calculates, for each pair of two consecutives preceding time window of the selected data, a difference between the values reached by the parameter respectively over each preceding time window of the pair of two consecutives preceding time windows.

In this case, two preceding time windows of a pair are temporally consecutive. In other words, if the preceding time windows of the selected data are ordered chronologically, two preceding time windows are consecutive if they follow each other. For example, if the time scale corresponds to one week, two consecutive preceding time windows are separated by exactly one week.

The difference is for example the absolute value of the difference between the values reached by the parameter respectively over each preceding time window of the pair of two consecutives preceding time windows.

In an example in which the time scale corresponds to one month and in which the processing unit 19 selects the data corresponding to the last four time windows, i.e. the last four days spaced from the day to come by one month, two months, three months and four months respectively, the processing unit 19 calculates three differences.

In fact, if these four days are ordered chronologically, the processing unit 19 calculates a first difference between the value reached by the parameter over the first day and the value reached by the parameter over the second day, a second difference between the value reached by the parameter over the second day and the value reached by the parameter over the third day and a third difference between the value reached by the parameter over the third day and the value reached by the parameter over the fourth day.

In the sub-step S23, the processing unit 19 assigns to the tested time scale a stability score characterizing the one or more calculated differences. The stability score assigned to the time scale depends for instance on a ratio of pair of two consecutive preceding time windows of the selected data for which the calculated difference is lower than or equal to a predetermined threshold. The predetermined ratio used to determine the stability can be equal to or different from the predetermined threshold used to determine the regularity ratio.

For example, we now consider that the tested time scale corresponds to one day and that the processing unit 19 selected the data corresponding to the last five days. The processing unit 19 has thus calculated the difference between the values reached by the parameter respectively over each preceding time window of each of the four pairs of consecutives days. Furthermore, if the parameter corresponds to the traveled distance, the predetermined threshold can be set to 2 kilometers. The stability score can then correspond to the proportion of these four pairs of consecutive days for which there is less than 2 kilometers difference between the distance traveled by the individual over the first day of a pair and the second day of the same pair.

In the sub-step S24, the processing unit 19 determines, according to the value of the local variable, whether all the time scales have been tested. Otherwise, the following time scale is selected to be tested according to the sub-steps S21, S22 and S23 as explained previously.

Finally, in the sub-step S25, the processing unit 19 selects the appropriate time scale based on the respective stability scores of the time scales of the at least two predetermined time scales.

If the stability score assigned to a time scale depends on a ratio of pair of two consecutive preceding time windows of the selected data for which the calculated difference is lower than or equal to a predetermined threshold, the appropriate time scale is for instance the one with the highest score.

The two embodiments previously described with reference to **FIG. 3** can also be combined. For example, it is possible to calculate, for each time scale, both the regularity score and the stability score and then to compare the scores of the time scales to select the appropriate time scale. Another possible combination is also to choose one or the other of the embodiments according to the time scale selected for the time window immediately preceding the upcoming time window.

In the following description of the method, reference is again made to **FIG. 2.**

In a step S3, the processing unit 19 retrieves data relating to one or more preceding time windows based on the selected appropriate time scale. Typically, the retrieved data relate to one or more preceding time windows spaced from the upcoming time window by a multiple of the period of time to which the selected appropriate time scale relates.

It must be noted that the retrieved data are not necessarily exactly the ones selected in sub-step S21 for testing the finally selected time scale. If the selected time scale corresponds to a week and if three days were selected in sub-step S21, the processing unit 19 can, in step S3, select six days, i.e. the same day as the upcoming day, but for the last six weeks.

In a step S4, the processing unit 19 calculates a success factor based on the comparison between the value reached by the parameter over one or more preceding time windows and the associated target value.

Indeed, one of the advantages of the invention is to take the past success or failure to reach the target value into account. For instance, when the parameter is the distance covered over each time window, i.e. each day, by the individual, the success factor makes it possible to take into account the individual's ability to succeed, the day before or the days before, in reaching the set goal, i.e. the determined target value. This success factor thus makes it possible to consider the individual's recent performance and his or her ability to achieve his or her objectives.

This success factor typically takes the form of a multiplicative coefficient greater than or equal to 1 when the individual has globally succeeded, over one or more days preceding the day to come, in reaching the set goal. Conversely, this multiplicative coefficient is less than 1 when the individual has globally failed, over one or more days preceding the day to come, to reach the set goal. The skilled person understands that this reasoning can be applied to any parameter, and not only the number of steps or the distance covered by an individual measured by a portable pedometer-type device sensitive to motion.

As an example, the value of the success factor can be determined as follows: the processing unit 19 selects the last three preceding time windows, so typically the last three days. For each of these last three days, the storage unit 17 stores the value reached by the parameter, for instance the number of steps or the distance traveled by an individual, as well as the target value determined for each of these three days. If the value of the parameter is greater than the associated target value for all three days, then the success factor is equal to 1.1 (or 110%). On the other hand, if the value of the parameter is lower than the associated target value for the three days considered, then the success factor is equal to 0.9 (or 90%). In the other cases, if the value of the parameter is higher than the associated target value only for one or two days, then the success factor is equal to 1 (or 100%).

Of course, it is possible to calculate the success factor based on only one or two preceding time windows or many more than three time windows. Similarly, it is possible to determine the value of the success factor in several ways.

Additionally, the success factor can be calculated based on a profile of the individual depending on physiological characteristics or data relating to the regular physical activity or health of the individual.

In a step S5, the processing unit 19 determines the target value for the upcoming time window based on the success factor and the value reached by the parameter over each preceding time window of the retrieved data. The retrieved data correspond here to the data retrieved during step S3.

The target value to be reached by the parameter over an upcoming time window j can be calculated as follows:

$$TV(j) = SF \times \frac{1}{k} \sum_{i=1}^{k} P(j-i)$$

where:

- j is the upcoming time window;
- TV(j) is the target value to be reached by the parameter over the upcoming time window;
- k is the number of preceding time window of the retrieved data;
- j-i is the i-th preceding time window;
- P(j-i) is the value reached by the parameter over the i-th preceding time window; and
- SF is the success factor.

In the previous formula, it must be noted that, if the time window corresponds to a day, the appropriate time scale corresponds to one day since the preceding time windows of the retrieved data are indexed j-i.

In the embodiment illustrated in **FIG. 2,** the step S5 comprises a preliminary sub-step S51 wherein the value reached by the parameter over each preceding time window of the retrieved data is weighted. Typically, the weighting of the value reached by the parameter over a given time window is greater than the weighting of the value reached by the parameter over a time window prior to the given time window.

This weighting sub-step S51 is particularly suitable when the target value is determined according to a linearly weighted moving average calculation.

For instance, the target value to be reached by the parameter over an upcoming time window j can be calculated as follows:

$$TV(j) = SF \times \sum_{i=1}^{k} w(j - 7i) \times P(j - 7i)$$

where:

- j is the upcoming time window;
- TV(j) is the target value to be reached by the parameter over the upcoming time window;
- k is the number of preceding time window of the retrieved data;
- j-7i is the i-th preceding time window;
- w(j-7i) is the weighting coefficient of the i-th preceding time window;
- P(j-7i) is the value reached by the parameter over the i-th preceding time window; and
- SF is the success factor.

In the previous formula, it must be noted that, if the time window corresponds to a day, the appropriate time scale corresponds to one week since the preceding time windows of the retrieved data are indexed j-7i.

Still referring to **FIG. 2,** the steps described below relate to a particular embodiment in which the target value to be reached by the parameter over the upcoming time window is also determined by learning.

In a step T1, the device 3 measures parameter values. As explained above, the parameter values can be measured in real time or at regular time intervals. These values are measured over one or more time windows and are transmitted by the communication module 11 of the device 3 to the communication module 15 of the server 5 to be stored in the storage unit 17. As previously explained, the device 3 is for example a portable pedometer-type device sensitive to motion and the measured parameter is the number of steps or the distance traveled by the individual wearing the device 3. The measured values can be displayed in real time or at regular time intervals by the screen 13 of the device 3.

As illustrated in **FIG. 2,** the measurements collected by the device 3 and transmitted to the server 5 are used to generate the data provided to the processing unit 19 in order to determine the target value to be reached by the parameter over the upcoming time window.

The skilled person understands here that this step T1 is not unique to the embodiment in which the target value is determined at least in part by learning and can therefore be implemented independently, if only to generate the data stored in the storage unit 17.

In a step T2, the processing unit 17, and more precisely the processor 23, generates a time series including values of the parameter. A time series is a series of data points indexed or listed in time order. In other words, this time series takes the form of a set of parameter values each associated with a time component which characterizes, for example, the instant at which the value was measured.

The time series can be updated with each new value reached by the parameter over a given time window. In other words, the time series is updated each time the device 3 measures a new value of the parameter and transmits this value to the server 5.

In an optional step T3, the processing unit 19 segments the time series into a plurality of groups of time windows according to a criterion related to the respective time components of the time windows.

The temporal components of the time windows allow to order them in time, i.e. to classify them chronologically. The temporal component of a time window allows to know, for example, if this time window is earlier or later than another time window, if the temporal component associated with the latter is known. Therefore, this criterion consists, for instance, in grouping consecutive time windows once they have been classified chronologically. According to an embodiment, each group comprises the same number of time windows.

When the parameter relates to the physical activity of an individual, for example the number of steps or the distance covered over each time window, a segmentation of the time series is particularly advantageous since the physical activity of an individual depends on external conditions like for example the season. Typically, the time series can be segmented into four groups so that each group corresponds to a season. This segmentation allows to individualize the data and to take potential drastic changes in the individual's daily life into account.

In a step T4, the processing unit 19 generates by learning at least one predictive model of the value of the parameter for a given time window as a function of a time component characterizing the considered time window.

It can be supervised or semi-supervised learning.

If the optional step T3 is implemented, a predictive model is generated by learning for each group of time windows. Conversely, if the time series is not segmented by the processing unit 19, only one predictive model can be generated in step T4.

In a step T5, the processing unit 19 applied the criterion to the time component of the upcoming time window in order to determine the corresponding group of time windows.

If we consider the previous example in which the parameter relates to the physical activity of an individual and in which the time series is for example segmented into four groups corresponding to the seasons, it appears that the principle of the step T5 is to determine, using the time component of the upcoming time window, therefore the day to come, to which season this upcoming day belongs. The temporal component thus makes it possible to determine to which group of the time series the upcoming day is closest.

In the above-mentioned step S5 illustrated in **FIG. 2,** the predictive model associated with said group of time windows is used to determine the target value for the upcoming time window.

In the absence of segmentation and therefore if only one predictive model has been generated, then it is not necessary to apply such a criterion and the predictive model can be used to determine, depending on the time component of the upcoming time window, the target value to be reached by the parameter on the upcoming time window.

Of course, the predictive model can be used alone to determine the target value but can also be used in combination with the embodiments detailed previously, i.e. with the weighting coefficients for the value reached by the parameter over one or more preceding time windows and with the data relating to the profile of the individual including physiological characteristics or data relating to the regular physical activity or health of the individual.

**Claims**

1. A computer-implemented method for determining a target value to be reached for a parameter over an upcoming time window comprising:

   - providing (S1) a set of data relating to preceding time windows and including, for each preceding time window, the value reached by said parameter over said preceding time window and the target value associated with said preceding time window;
   - selecting (S2) an appropriate time scale among at least two predetermined time scales;
   - retrieving (S3) data relating to one or more preceding time windows based on said time scale;
   - calculating (S4) a success factor based on the comparison between the value reached by said parameter over one or more preceding time windows and the associated target value; and
   - determining (S5) the target value for the upcoming time window based on said success factor and the value reached by said parameter over each preceding time window of the retrieved data.

2. The method of claim 1, wherein the value of said parameter is measured by a portable device (3) adapted to be worn by an individual.

3. The method of claim 2, the portable device being a pedometer-type device sensitive to motion, wherein the parameter quantifies the intensity or quality of an individual's physical activity, such as a number of steps, a distance traveled by an individual, a number of calories burned or the duration of said physical activity.

4. The method of claim 3, wherein the success factor is calculated based on a profile of said individual depending on physiological characteristics or data relating to a regular physical activity or health of said individual.

5. The method of one of the previous claims, wherein the selection of the appropriate time scale depends on the variations in the parameter value over one or more preceding time windows.

6. The method of claim 5, wherein each time scale of the at least two predetermined time scales relates to a period of time, and wherein selecting the appropriate time scale comprises:

   - for each time scale of the at least two predetermined time scales:

     • selecting (S21) data relating to preceding time windows spaced from the upcoming time window by a multiple of the period of time to which said time scale relates;
     • calculating (S22), for each pair of two consecutive preceding time windows of the selected data, a difference between the values reached by the parameter respectively over each preceding time window of said pair of two consecutives preceding time windows;
     • assigning (S23) to said time scale a stability score characterizing the one or more calculated differences; and

   - selecting (S25) the appropriate time scale based on the respective stability scores of the time scales of the at least two predetermined time scales.

7. The method of claim 6, wherein the stability score assigned to a time scale depends on a ratio of pair of two consecutive preceding time windows of the selected data for which the calculated difference is lower than or equal to a predetermined threshold.

8. The method of one of claims 5 to 7, wherein each time scale of the at least two predetermined time scales relates to a period of time, and wherein selecting the appropriate time scale comprises:

- for each time scale of the at least two predetermined time scales:

• selecting data relating to one or more preceding time windows spaced from the upcoming time window by a multiple of the period of time to which said time scale relates;
• calculating, for each preceding time window of the selected data, a difference between the value reached by the parameter over said preceding time window and the target value associated with said preceding time window;
• assigning to said time scale a regularity score characterizing the one or more calculated differences; and

- selecting the appropriate time scale based on the respective regularity scores of the time scales of the at least two predetermined time scales.

9. The method of claim 8, wherein the regularity score assigned to a time scale depends on a ratio of preceding time windows of the selected data for which the calculated difference is lower than or equal to a predetermined threshold.

10. The method of one of the previous claims, wherein each time scale of the at least two predetermined time scales relates to a period of time, and wherein the retrieved data relate to one or more preceding time windows spaced from the upcoming time window by a multiple of the period of time to which the selected time scale relates.

11. The method of one of the previous claims, wherein determining the target value for the upcoming time window comprises weighting (S51) of the value reached by the parameter over each preceding time window of the retrieved data so that the weighting coefficient of the value reached by the parameter over a given time window is greater than or equal to the weighting coefficient of the value reached by the parameter over a time window prior to said given time window.

12. The method of one of the previous claims, further comprising:

- generating (T2) a time series including values of the parameter, said time series being updated with each new value reached by said parameter over a given time window; and
- generating (T4) by learning at least one predictive model of the value of the parameter for a given time window as a function of a time component characterizing said time window; wherein the target value for the upcoming time window is determined using said at least one predictive model.

13. The method of claim 12, wherein the time series is segmented (T3) into a plurality of groups of time windows according to a criterion related to the respective time components of said time windows and a predictive model is generated by learning for each group of time windows, and wherein determining the target value for the upcoming time window comprises:

- applying (T5) said criterion to the time component of the upcoming time window in order to determine the corresponding group of time windows; and
- using the predictive model associated with said group of time windows to determine the target value for the upcoming time window.

14. Computer program comprising instructions for implementing the method of one of the previous claims, when said instructions are executed by at least one processor (9, 23).

15. A system (1) for determining a target value to be reached for a parameter over an upcoming time window comprising:

• a communication module (15) arranged to receive a set of data relating to a plurality of preceding time windows;
• a storage unit (17) arranged to store the received set of data, the data relating to a preceding time window including the value reached by said parameter over said preceding time window and the associated target value; and
• a processing unit (19) configured to:

- select an appropriate time scale among at least two predetermined time scales;
- retrieve, from the storage unit, data relating to one or more preceding time windows based on said time scale;
- calculate a success factor based on the comparison between the value reached by said parameter over one or more preceding time windows and the associated target value; and
- determine the target value for the upcoming time window based on said success factor and the value reached by said parameter over each preceding time window of the selected data.

FIG. 1

T1 — MEAS

T2 — GENERAT

T3 — SEGM

T4 — PREDICT MOD

T5 — APPL

PROV SET — S1

SELEC TS — S2

RETR DAT — S3

CALC SUCC — S4

WGHT — S51

DET TRGT — S5

# FIG. 2

S2

i = 1

S21 — SELEC DAT

S22 — CALC DIFF

S23 — ASGN

S24 — i < N ?

OK

i ⇐ i + 1

KO

S25 — SELECT TS

FIG. 3

Europäisches
Patentamt

European
Patent Office

Office européen
des brevets

**EUROPEAN SEARCH REPORT**

Application Number

EP 21 30 5812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/135592 A1 (OHNEMUS PETER [CH] ET AL) 15 May 2014 (2014-05-15)<br>* paragraph [0006] *<br>* paragraph [0068] - paragraph [0070] *<br>* paragraph [0072] *<br>* paragraph [0082] - paragraph [0084] *<br>* Facsimile page 12 to page 13 * | 1-15 | INV.<br>G16H50/20<br>G16H50/30<br>A61B5/11 |
| X | US 2016/371998 A1 (FAZEEL MUHAMMED [US]) 22 December 2016 (2016-12-22)<br>* paragraph [0014] *<br>* paragraph [0040] *<br>* paragraph [0072] - paragraph [0073] *<br>* paragraph [0081] *<br>* paragraph [0083] * | 1-15 | |
| X | Graham Bower: "10 hidden Activity app features that will take your fitness to the next level",<br>,<br>16 April 2019 (2019-04-16), XP055868737,<br>Retrieved from the Internet:<br>URL:https://www.cultofmac.com/616240/hidden-activity-app-features/<br>[retrieved on 2021-12-02]<br>* the whole document * | 1-15 | TECHNICAL FIELDS SEARCHED (IPC)<br><br>G16H<br>A61B |
| X | US 10 602 964 B2 (KONINKLIJKE PHILIPS NV [NL]) 31 March 2020 (2020-03-31)<br>* claims 1,4,9-10 *<br>* paragraph [0006] - paragraph [0007] *<br>* paragraph [0026] - paragraph [0027] *<br>* paragraph [0034] *<br>* paragraph [0038] *<br>* paragraph [0050] * | 1-15 | |

The present search report has been drawn up for all claims

| Place of search | Date of completion of the search | Examiner |
|---|---|---|
| Munich | 2 December 2021 | Martínez Cebollada |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 5812

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2021

| Patent document cited in search report | | Publication date | Patent family member(s) | | Publication date |
|---|---|---|---|---|---|
| US 2014135592 | A1 | 15-05-2014 | US  2014135592 A1<br>WO 2014091311 A2 | | 15-05-2014<br>19-06-2014 |
| US 2016371998 | A1 | 22-12-2016 | NONE | | |
| US 10602964 | B2 | 31-03-2020 | NONE | | |

EPO FORM P0459

For more details about this annex : see Official Journal of the European Patent Office, No. 12/82